(19) Europäisches Patentamt
European Patent Office
Office européen des brevets

(11) Numéro de publication: **0 055 196**
**B1**

# (12) FASCICULE DE BREVET EUROPEEN

(45) Date de publication du fascicule du brevet: **18.04.84**

(51) Int. Cl.$^3$: **C 07 C 37/00**, C 07 C 39/27

(21) Numéro de dépôt: **81420185.1**

(22) Date de dépôt: **18.12.81**

## (54) Procédé de préparation de phénols métachlorés.

(30) Priorité: **24.12.80 FR 8027936**

(43) Date de publication de la demande:
**30.06.82 Bulletin 82/26**

(45) Mention de la délivrance du brevet:
**18.04.84 Bulletin 84/16**

(84) Etats contractants désignés:
**BE DE FR GB IT LU NL**

(56) Documents cités:
**FR - A - 2 209 738**
**FR - A - 2 285 355**

**CHEMICAL ABSTRACTS, vol. 86, no. 7, 14 février 1977, réf. no. 43346k, page 503 COLUMBUS OHIO (US)**

(73) Titulaire: **RHONE-POULENC SPECIALITES CHIMIQUES, "Les Miroirs" 18, Avenue d'Alsace, F-92400 Courbevoie (FR)**

(72) Inventeur: **Cordier, Georges, 50, Chemin des Hermières, F-69340 Francheville (FR)**

(74) Mandataire: **Vignally, Noel et al, RHONE-POULENC RECHERCHES Centre de recherches de Saint Fons Service Brevets Boîte postale 62, F-69190 Saint Fons (FR)**

EP 0 055 196 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

Procedé de préparation de phenols métachlores

La présente invention a pour objet un procédé de préparation de phénols comportant un atome de chlore sur l'une au moins des positions méta par rapport à l'hydroxyle phénolique, par hydrodéchloration de chlorophénols plus fortement chlorés.

Par la suite, pour des raisons de commodité, on désignera par l'expression «phénols métachlorés» les phénols comportant un atome de chlore sur l'une au moins des positions méta.

Les phénols métachlorés, et en particulier le chloro-3 phénol et le dichloro-3,5 phénol, sont des composés qui revêtent un très grand intérêt industriel comme intermédiaires en synthèse organique.

Diverses méthodes de préparation des phénols métachlorés ont été proposées. On distingue notamment des méthodes engendrant la fonction phénol sur des composés aromatiques chlorés (par exemple l'hydrolyse alcaline des polychlorobenzènes; la nitration des chloro-3 et dichloro-3,5 benzènes suivie de la réduction du groupe nitro en groupe amino, de la diazotation de ce dernier et de la décomposition du sel de diazonium); des méthodes de chloration de phénols et des méthodes de déchloration de polychlorophénols. Cette dernière méthode présente un très grand intérêt industriel en raison de la disponibilité des polychlorophénols dont certains sont des produits courants et d'autres des sous-produits d'intérêt limité qu'il import de valoriser.

C'est ainsi, par exemple, que l'on obtient des tri- et tétrachlorophénols isomères dont certains comportent un ou deux atomes de chlore en position méta par rapport à l'hydroxyle phénolique lors de la préparation du tétrachloro-2,3,4,6 phénol et du pentachlorophénol par chloration du dichloro-2,6 phénol sous-produit de la préparation du dichloro-2,4 phénol. Ces divers polychlorophénols constituent des matières premières de choix pour la préparation des phénols métachlorés par déchloration. Une méthode d'élimination des atomes de chlore excédentaires consiste à soumettre les polychlorophénols à une hydrogénation en phase vapeur ou en phase liquide en présence d'un catalyseur. Pour des raisons de simplification, on désignera par la suite par «hydrodéchloration» la déchloration des polychlorophénols par hydrogénation.

Le problème essentiel posé par l'hydrodéchloration des polychlorophénols en chloro-3 ou dichloro-3,5 phénols réside dans l'élimination sélective des atomes de chlore en position 2 et/ou 4 et/ou 6 par rapport à l'hydroxyle phénolique. On a proposé divers procédés d'hydrodéchloration des polychlorophénols mais aucun ne s'est révélé jusqu'ici pleinement satisfaisant.

Ainsi, dans le brevet américain 2 803 669 délivré le 20 août 1957 on a décrit un procédé d'hydrodéchloration de polychlorophénols en phase vapeur par passage d'un mélange gazeux hydrogène/polychlorophénols sur un catalyseur à base d'halogénures cuivreux (chlorure cuivreux par exemple) déposés sur de l'alumine, maintenu à température élevée (350 à 550°C). Appliqué à l'hydrodéchloration du tétrachloro-2,3,4,6 phénol, ce procédé n'a pas permis d'obtenir une élimination sélective des atomes de chlore en position 2, 4 et 6 par rapport à l'hydroxyle phénolique. La masse réactionnelle résultant de l'hydrogénation est constituée pour l'essentiel de dichloro-2,4 et de dichloro-2,6 phénols.

Dans la demande de brevet français No. 73-43.484 publiée sous le No. 2 209 738, on a proposé un procédé de préparation de phénols métahalogénés par déshalogénation des polyhalogénophénols par hydrogénation en phase liquide à température élevée, en présence d'un catalyseur comportant soit un ou plusieurs sulfures ou polysulfures de fer, de nickel ou de cobalt, soit un métal noble tel que le palladium ou le platine associé à un dérivé du soufre. La réaction est de préférence conduite en présence d'une base telle que les hydroxydes ou les carbonates alcalins pour neutraliser les hydracides engendrés par la réaction au fur et à mesure de leur formation. Bien que ce procédé se montre très sélectif vis-à-vis de la formation des phénols métachlorés, il présente l'inconvénient de devoir être mis en œuvre en présence d'une base et en particulier d'une base alcaline dans des conditions de température (cette dernière doit être comprise de préférence entre 180 et 330°C) favorables à la formation d'halogénodioxines et en particulier de polychlorodioxines dont on sait que certaines présentent une toxicité élevée. Un tel inconvénient enlève en pratique tout intérêt industriel à ce procédé. En définitive l'industrie est encore à la recherche d'un procédé sélectif d'obtention de phénols métachlorés par hydrodéchloration de polychlorophénols s'affranchissant de la présence de bases alcalines. La présente invention se propose précisément de mettre à la disposition de l'industrie un procédé d'hydrodéchloration sélective des polychlorophénols exempt des inconvénients précités.

Plus spécifiquement, la présente invention a pour objet un procédé d'obtention sélective de chlorophénols comportant un atome de chlore sur l'une au moins des positions méta par rapport à l'hydroxyle phénolique, par hydrogénation à chaud en phase liquide, en présence d'un catalyseur à base d'un métal noble du groupe VIII de la classification périodique, de polychlorophénols de formule générale (I):

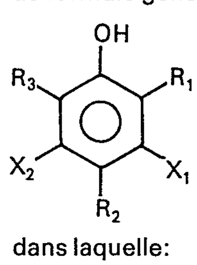

(I)

dans laquelle:

$X_1$ et $X_2$, identiques ou différents représentent un atome de chlore, un atome d'hydrogène, un radical alkyle, aryle, arylalkyle, alkyloxy, aryloxy, l'un au moins des symboles $X_1$ et $X_2$ représentant un atome de chlore,

$R_1$, $R_2$, et $R_3$, identiques ou différents, représentent un atome de chlore, un atome d'hydrogène, un radical alkyle, un radical aryle, arylalkyle, un radical alkyloxy, aryloxy, l'un au moins des symboles $R_1$, $R_2$, et $R_3$ représentant un atome de chlore, caractérisé en ce que la réaction est conduite dans un milieu solvant acide au moins partiellement aqueux contenant des ions halogénures.

Plus spécifiquement ceux des radicaux $X_1$, $X_2$, $R_1$, $R_2$, et $R_3$ qui ne symbolisent pas un atome de chlore représentent un radical alkyle comportant de 1 à 10 atomes de carbone et de préférence de 1 à 4 atomes de carbone comme les radicaux méthyle, éthyle, n-propyle, isopropyle, n-butyle, isobutyle, t-butyle; un radical phényle; un radical benzyle; un radical alkyloxy comportant de 1 à 10, et de préférence de 1 à 4 atomes de carbone comme les radicaux méthoxy, éthoxy, n-propyloxy, isopropyloxy, n-butyloxy; le radical phénoxy.

Parmi les ions halogénures convenant à la mise en œuvre du procédé selon l'invention, on peut citer les ions chlorure, bromure et iodure qui peuvent être utilisés seuls ou en combinaison. On utilise de préférence les ions bromure et chlorure seuls ou les associations $Cl^-/I^-$ et $Cl^-/Br^-$.

Le milieu solvant dans lequel est effectuée la réaction peut être constitué par l'eau seule ou par un mélange en toutes proportions d'eau et d'un ou plusieurs solvants organiques, liquides et inertes dans les conditions de la réaction. Il n'est pas nécessaire que ce ou ces solvants soient miscibles à l'eau; leur rôle consiste essentiellement à dissoudre les polychlorophénols. Comme exemples de solvants on peut citer: des hydrocarbures aliphatiques tels que l'octane, l'hexane; des hydrocarbures cycloaliphatiques tels que le cyclohexane; des hydrocarbures aromatiques tels que le benzène, le toluène, les xylènes; des hydrocarbures aromatiques chlorés tels que le monochlorobenzène et les polychlorobenzènes.

Parmi ces solvants le monochlorobenzène et les polychlorobenzènes sont particulièrement intèressants.

Essentiellement en raison de leurs points d'ébullition, on utilise plus préférentiellement les dichlorobenzènes et les trichlorobenzènes, lorsque le milieu solvant de la réaction est constitué par un mélange d'eau et de solvant organique.

Le rapport volumique eau/solvant organique n'est pas critique; l'eau peut représenter par exemple de 5% à 100% du volume total du milieu solvant. Le plus souvent l'eau représente de 50 à 100% du volume total du milieu solvant.

Dans ce qui suivra on emploiera par commodité les termes solution aqueuse pour désigner le milieu solvant dans lequel s'effectue la réaction, mais il est bien entendu que ces termes recouvrent également les solutions à base de mélanges eau/solvant organique tels que définis précédemment. Les concentrations des différents composés seront exprimées non par rapport à l'eau seule, mais par rapport au volume global du milieu solvant.

Les solutions aqueuses acides contenant des ions halogénures utilisées comme milieu réactionnel pour la mise en œuvre du procédé selon l'invention peuvent être obtenues par addition à des solutions aqueuses d'acides minéraux forts non halogénés tels que les acides sulfurique ou phosphorique, d'un ou plusieurs composés libérant des ions halogénures en solution aqueuse, dénommés ci-après par commodité «vecteurs d'halogénures». Parmi ces derniers, on peut citer les halogénures (chlorures, bromures, iodures) alcalins ou alcalino-ferreux, les halogénures d'ammonium, les halogénures d'ammonium quaternaire, les halohydrates d'amines. Plus simplement on a recours de préférence à des solutions aqueuses d'acide chlorhydrique, d'acide bromhydrique et d'acide iodhydrique qui fournissent à la fois au milieu réactionnel l'acidité et les ions chlorure, bromure et iodure nécessaires au déroulement de l'hydrodéchloration.

L'acidité du milieu peut varier dans de larges limites. De préférence la concentration en protons de la solution aqueuse est d'au moins 0,5 ions $H^+$ par litre. Il n'y a pas de valeur supérieure critique de cette concentration, bien qu'une trop grande acidité du milieu réactionnel ne soit pas désirée afin de limiter la corrosion de l'appareillage. En général la concentration en protons ne dépasse pas 15 ions $H^+$ par litre et, lorsque la nature de l'acide utilisé le permet, 8 ions $H^+$ par litre. En pratique la concentration en protons est de préférence comprise entre 1 et 6 ions $H^+$ par litre.

Il n'y a pas en général de valeur maximale critique pour la concentration en ions halogénures; cependant pour des raisons pratiques, il n'est pas nécessaire d'aller au delà d'une concentration de 15 ions-g/l d'halogénure. Par contre, on a constaté que la concentration en ions halogénures de la phase aqueuse ne pouvait être abaissée en deçà d'une valeur minimale si l'on veut conserver à l'hydrodéchloration son caractère sélectif. Cette valeur minimale critique dépend de la nature de l'halogénure. Ainsi lorsque l'ion halogénure est l'ion bromure la concentration en $Br^-$ peut être abaissée jusqu'à 2 ions-g/l et de préférence 4 ion-g/l tout en conservant à l'hydrodéchloration une excellente sélectivité. Dans le cas des ions chlorures, la concentration est de préférence au moins égale à 8 ions $Cl^-$ par litre et plus préférentiellement encore au moins égale à 10 ions $Cl^-$ par litre. Dans ce cas on constate que si l'on a recours à l'acide chlorhydrique comme fournisseur de protons et d'ions chlorures, la concentration en protons est dépendante de la concentration minimale en ions chlorures. La mise en œuvre de concentrations élevées d'ions chlorure se traduit «ipso facto» par une concentration en protons plus élevée que celle strictement nécessaire à un bon déroulement de la réaction. Dans ces conditions, l'emploi de l'acide chlorhydrique, plus

avantageux au plan économique que celui de l'acide bromhydrique présente un inconvénient notable du point de vue corrosion. Or il s'avère intéressant au plan industriel de limiter l'acidité du milieu de façon à limiter le plus possible la corrosion. On a trouvé, et celà constitue un autre aspect de la présente invention, que l'acide chlorhydrique peut être utilisé à des concentrations inférieures à 8 moles par litre, et en particulier à des concentrations fournissant au milieu une acidité se situant entre les limites données plus haut sans que le bon déroulement de la réaction en soit affecté, à la condition d'utiliser conjointement des composés fournissant des ions halogénures au milieu réactionnel sans modifier ou en modifiant peu l'acidité de ce milieu.

Parmi les composés qui conviennent à cet égard et que l'on nommera ci-après «vecteurs d'halogénures», on peut citer les halogénures alcalins ou alcalino-terreux tels que les chlorures, bromures et iodures de sodium, de potassium et de lithium; les chlorures, iodures, bromures d'ammonium; les chlorures, iodures, bromures d'ammonium quaternaires tel que les chlorures, iodures, bromures de tétraéthylammonium; les halohydrates d'amines. On peut également avoir recours aux acides bromhydrique et iodhydrique qui peuvent être mis en œuvre en faible quantité et ne modifient pas sensiblement l'acidité du milieu. De préférence on fait appel aux halogénures alcalins et plus préférentiellement encore aux bromures et iodures alcalins.

La quantité de vecteur d'halogénures utilisé conjointement à l'acide chlorhydrique dépend essentiellement de la nature de l'ion halogénure. Ainsi lorsqu'on utilise un chlorure cette quantité est calculée pour que la concentration totale en ions chlorures de la solution aqueuse acide soit au moins égale à 8 ions chlorures par litre. Par concentration totale en ions chlorure on entend la concentration en ions chlorure provenant de l'acide chlorhydrique d'une part et du vecteur d'ions chlorures utilisé d'autre part. En pareil cas la quantité d'acide chlorhydrique est calculée de façon à ce que la concentration en protons soit comprise entre les limites indiquées précédemment et en particulier entre 1 et 6 ions $H^+$ par litre, le complément d'ions chlorures nécessaire pour atteindre la valeur minimale de la concentration en ions $Cl^-$ étant fournie par le vecteur d'ions chlorures mis en œuvre. Lorsqu'on fait appel à des vecteurs d'iodures ou à des vecteurs d'ions bromures les quantités utilisées pourront être notablement inférieures à celles des vecteurs d'ions chlorures. En effet, on a constaté que des concentrations en ions $I^-$ aussi faibles que $1.10^{-6}$ ions $I^-$ par litre et en ions $Br^-$ aussi faibles que $1.10^{-2}$ $Br^-$ par litre permettent d'obtenir de bons résultats. De préférence la concentration en ions $I^-$ dans le milieu réactionnel est au moins égale à $1.10^{-4}$ ion $I^-$ par litre et la concentration en ions bromures à au moins 0,1 ion $Br^-$ par litre. Il n'y a pas de limite supérieure critique des concentrations en ions halogénures comme celà a été indiqué précédemment. Néanmoins, pour des raisons pratiques, il n'est pas nécessaire de dépasser 1 ion $I^-$ par litre et 10 ions $Br^-$ par litre et de préférence 6 ions $Br^-$ par litre.

On pourrait, sans sortir du cadre de la présente invention, faire appel à l'emploi conjoint d'acide chlorhydrique et de deux ou plusieurs vecteurs d'ions halogénures mais celà n'apporterait pas d'avantage particulier.

La température de la réaction est généralement comprise entre 50 et 350°C et, de préférence entre 100 et 250°C.

La pression partielle d'hydrogène peut varier dans de larges limites et être supérieure, inférieure ou égale à la pression atmosphérique. Plus spécifiquement la pression partielle d'hydrogène est comprise entre 0,1 et 60 bars et, de préférence entre 0,5 et 50 bars. Ont pourrait avoir recours à des pressions supérieurs à 60 bars mais sans que celà se traduise par des avantages particuliers. La pression totale à laquelle s'effectue la réaction dépend essentiellement des conditions de température, de la volatilité de l'acide mis en œuvre dans ces conditions et de la valeur de la pression partielle d'hydrogène. Il va de soi que la pression totale doit être suffisante pour maintenir le milieu réactionnel liquide et/ou la concentration en acide de la phase aqueuse dans les limites précitées.

Les métaux nobles constituant la base des catalyseurs utilisés dans l'invention sont principalement des métaux du groupe VIII de la classification périodique tels que le ruthénium, le rhodium, le palladium, l'osmium, l'iridium et le platine; le palladium est le métal préféré. Le métal peut être à l'état métallique ou à l'état de composé chimique; généralement on préfère que le métal soit mis en œuvre à l'état métallique car, dans les conditions opératoires, les composés ont tendance à être réduit à l'état métallique. Le catalyseur peut être supporté ou non supporté. Comme support du catalyseur, on peut utiliser tout support connu en soi pour supporter des catalyseurs, pour vu que ce support soit résistant à l'eau et aux acides; comme support convenant plus particulièrement, on peut citer le noir de carbone, la silice, le sulfate de baryum; le noir de carbone est un support préféré. Le catalyseur ainsi que son support sont avantageusement sous forme finement divisée; des surfaces spécifiques supérieures à 100 $m^2/g$ conviennent généralement bien.

La quantité de catalyseur mise en œuvre est telle que la proportion pondérale de métal noble du catalyseur par rapport au composé de formule (I) à traiter est généralement comprise entre 0,01 et 10%, de préférence entre 0,1 et 5%.

Comme exemples de polychlorophénols de formule (I) qui peuvent être utilisés comme matières premières dans le procédé conforme à la présente invention, on peut citer:

- le dichloro-2,3 phénol
- le dichloro-2,5 phénol
- le dichloro-3,4 phénol
- le trichloro-2,3,4 phénol

- le trichloro-2,3,6 phénol
- le trichloro-2,4,5 phénol
- le trichloro-2,3,5 phénol
- le trichloro-3,4,5 phénol
- le tétrachloro-2,3,4,6 phénol
- le tétrachloro-2,3,4,5 phénol
- le tétrachloro-2,3,5,6 phénol
- le pentachlorophénol
- le trichloro-2,3,4 méthyl-6 phénol
- le dichloro-2,3 méthyl-6 phénol
- le tétrachloro-2,3,4,6 méthyl-5 phénol
- le dichloro-2,3 méthyl-4 phénol
- le tétrachloro-2,3,5,6 méthyl-4 phénol
- le dichloro-2,5 diméthyl-3,4 phénol
- le dichloro-2,5 éthyl-4 phénol
- le dichloro-2,5 propyl-4 phénol
- le dichloro-2,5 t-butyl-4 phénol
- le trichloro-3,4,6 benzyl-2 phénol
- le dichloro-3,4 méthoxy-2 phénol
- le dichloro-3,6 méthoxy-2 phénol
- le dichloro-4,5 méthoxy-2 phénol
- le dichloro-5,6 méthoxy-2 phénol
- le trichloro-3,4,6 méthoxy-2 phénol
- le trichloro-3,4,5 méthoxy-2 phénol
- le tétrachloro-3,4,5,6 méthoxy-2 phénol
- le dichloro-4,5 méthoxy-3 phénol
- le dichloro-5,6 méthoxy-3 phénol
- le dichloro-2,5 méthoxy-3 phénol
- le trichloro-4,5,6 méthoxy-3 phénol
- le trichloro-2,3,6 méthoxy-3 phénol
- le dichloro-4,5 phénoxy-2 phénol
- le tétrachloro-2,3,5,6 phénoxy-4 phénol
- le dichloro-3,4 éthoxy-2 phénol
- le trichloro-3,4,5 éthoxy-2 phénol
- le dichloro-3,4 phényl-2 phénol
- le trichloro-3,5,6 phényl-2 phénol.

En pratique on fait appel de préférence aux di- et trichlorophénols.

Parmi les phénols comportant un atome de chlore sur l'une au moins des positions en méta par rapport à l'hydroxyle phénolique qui peuvent être préparés par le procédé selon la présente invention, on peut citer:

- le chloro-3 phénol
- le dichloro-3,5 phénol
- le chloro-3 méthyl-6 phénol
- le chloro-3 méthyl-5 phénol
- le chloro-3 méthyl-4 phénol
- le dichloro-3,5 méthyl-4 phénol
- le chloro-5 diméthyl-3,4 phénol
- le dichloro-3,5 éthyl-4 phénol
- le dichloro-3,5 propyl-4 phénol
- le dichloro-3,5 t-butyl-4 phénol
- le chloro-3 benzyl-2 phénol
- le chloro-3 méthoxy-2 phénol
- le chloro-3 méthoxy-6 phénol
- le dichloro-3,5 méthoxy-2 phénol
- le chloro-3 méthoxy-5 phénol
- le chloro-3 phénoxy-6 phénol
- le dichloro-3,5 phénoxy-6 phénol
- le chloro-3 éthoxy-2 phénol
- le chloro-3 phényl-2 phénol.

Le procédé selon l'invention peut être mis en œuvre de façon continue ou discontinue. En fin de réaction le catalyseur est séparé par filtration et peut être recyclé tel quel dans une nouvelle opération d'hydrodéchloration. Les métachlorophénols formés peuvent être séparés du milieu réactionnel par extraction à l'aide d'un solvant organique non miscible à l'eau puis récupérés par distillation après élimination du solvant d'extraction.

Les exemples suivants illustrent l'invention et montrent comment elle peut être mise en pratique.

Exemple 1:
Dans un autoclave de 250 ml en acier inoxydable comportant un revêtement intérieur en tantale, équipé d'un système d'agitation, on charge:

1 g de dichloro-3,4 phénol
100 ml d'une solution aqueuse d'acide chlorhydrique 12 N
0,14 g d'un catalyseur au palladium déposé sur un charbon actif de surface spécifique 1000 $m^2.g^{-1}$ contenant 5% en poids de palladium métal (soit 0,007 g de palladium).

On porte le contenu de l'autoclave à 190°C après avoir fermé ce dernier, puis on introduit de l'hydrogène jusqu'à ce que la pression totale soit de 65 bars et maintient ces conditions pendant 5 heures. On refroidit ensuite le contenu de l'autoclave, le dégaze puis le soutire. On sépare le catalyseur de la phase aqueuse. Les chlorophénols sont ensuite extraits de la phase aqueuse par 300 ml d'éther. Le catalyseur est lavé par 3 fois 20 ml d'éther pour en extraire les chlorophénols qu'il contient. On réunit les extraits éthérés puis élimine l'éther par distillation et dose et identifie les chlorophénols présents dans le résidu de distillation par chromatographie en phase vapeur.

Les résultats de l'analyse montrent que la totalité du dichloro-3,4 phénol a été transformée (taux de transformation (TT): 100%). On a identifié dans le résidu de distillation:

du chloro-3 phénol: rendement par rapport au dichloro-3,4 phénol chargé (RR) = 93,3%
du phénol: RR = 6,7%.

Exemple 2:
On opère comme à l'exemple 1 en remplaçant l'acide chlorhydrique par une solution 6N d'acide bromhydrique, et en chargeant de l'hydrogène jusqu'à obtention d'une pression totale de 25 bars pour une température de 190°C. On maintient 90 mn dans ces conditions.

De cette façon on obtient le chloro-3 phénol avec un rendement de 100% par rapport au dichloro-3,4 phénol chargé (TT = 100%).

Exemple 3:
On opère comme à l'exemple 1 en remplaçant la solution aqueuse d'acide chlorhydrique 12 N par 100 ml d'une solution aqueuse d'HCl 6N et en

chargeant en outre 0,022 g d'iodure de potassium (soit 0,0013 mole par litre de solution).

La durée de la réaction est de 260 mn. Après séparation et dosage des produits de la réaction, on constate que le taux de transformation du dichlorophénol est de 100%, le RR en chloro-3 phénol de 95% le RR enphénol de 5%.

A titre comparatif, on a répété l'expérience précédente en omettant de charger l'iodure de potassium mais toutes choses étant égales par ailleurs. Dans ces conditions on constate qu'il ne se forme pas de chloro-3 phénol mais principalement du cyclohexanol et de la cyclohexanone.

Exemple 4:

On opère comme à l'exemple 3 mais en remplaçant le dichloro-3,4 phénol par le dichloro-2,5 phénol et en chargeant 0,11 g d'iodure de potassium par litre de solution aqueuse d'acide chlorhydrique.

Dans ces conditions le TT du dichlorophénol est de 93% et le rendement en chloro-3 phénol par rapport au dichlorophénol transformé (RT) de 100%.

Exemple 5:

Dans un autoclave de 250 ml en acier inoxydable comportant un revêtement intérieur en tantale, équipé d'un système d'agitation, on charge:

2,6 g de pentachlorophénol
0,5 g de catalyseur Pd/charbon à 5% de métal (identique à celui de l'exemple 1)
15 ml de dichloro-1,2 benzène
90 ml d'acide chlorhydrique aqueux 12 N
1,2 ml d'acide iodhydrique aqueux 8 N
30 ml d'eau

L'autoclave est fermé, purgé de l'air qu'il contient par trois fois 5 bars d'azote, puis par trois fois 5 bars d'hydrogène. L'autoclave est préssurisé avec 20 bars d'hydrogène (à température ambiante). On chauffe à 210°C et on laisse réagir à cette température pendant 21 heures 30 minutes. Après refroidissement et dégazage on traite la masse réactionnelle comme dans l'exemple 1.

On obtient les résultats suivants:

TT du pentachlorophénol: 100%
RR en dichloro-3,5 phénol: 85%
RR en trichloro-2,3,5 phénol: 5,1%
RR en tétrachloro-2,3,5,6 phénol: 9,4%

**Revendications**

1. Procédé d'obtention sélective de chlorophénols comportant un atome de chlore sur l'une au moins des positions méta par rapport à l'hydroxyle phénolique, par hydrogénation à chaud en phase liquide, en présence d'un catalyseur à base d'un métal noble du groupe VIII de la classification périodique, de polychlorophénols de formule générale (I):

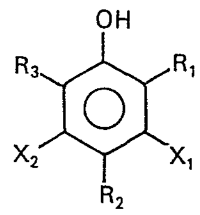

(I)

dans laquelle:

$X_1$ et $X_2$, identiques ou différents représentent un atome de chlore, un atome d'hydrogène, un radical alkyle, aryle, arylalkyle, alkyloxy, aryloxy, l'un au moins des symboles $X_1$ et $X_2$ représentant un atome de chlore,

$R_1$, $R_2$, et $R_3$, identiques ou différents, représentent un atome de chlore, un atome d'hydrogène, un radical alkyle, un radical aryle, arylalkyle, un radical alkyloxy, aryloxy, l'un au moins des symboles $R_1$, $R_2$, et $R_3$ représentant un atome de chlore, caractérisé en ce que la réaction est conduite dans un milieu solvant acide au moins partiellement aqueux contenant des ions halogénures.

2. Procédé selon la revendication 1, caractérisé en ce que le milieu solvant au moins partiellement aqueux est constitué par de l'au ou par un mélange eau/solvant organique comportant au moins 5% en volume d'eau et de préférence au moins 50% en volume d'eau.

3. Procédé selon l'une des revendications 1 ou 2, caractérisé en ce que ceux des radicaux $X_1$, $X_2$, $R_1$, $R_2$ et $R_3$ qui ne symbolisent pas un atome de chlore représentent un radical alkyle comportant de 1 à 10 atomes de carbone; un radical phényle; un radical benzyle; un radical alkyloxy comportant de 1 à 10 atomes de carbone; un radical phénoxy.

4. Procédé selon l'une qualconque des revendications 1 à 3, caractérisé en ce que la concentration en protons de la solution aqueuse acide est d'au moins 0,5 ion-g/l.

5. Procédé selon l'une quelconque des revendications 1 à 4, caractérisé en ce que la concentration en protons de la solution aqueuse acide est au plus égale à 15 ions-g/l.

6. Procédé selon l'une quelconque des revendications 1 à 5, caractérisé en ce que la concentration en ions halogénures de la solution aqueuse acide est d'au moins 2 ions-g/l.

7. Procédé selon l'une quelconque des revendications 1 à 6, caractérisé en ce que la concentration en ions halogénures de la solution aqueuse acide est au plus égale à 15 ions-g/l.

8. Procédé selon l'une qualconque des revendications 1 à 7, caractérisé en ce que la solution aqueuse acide contenant des ions halogénures est une solution aqueuse d'un hydracide pris dans le groupe formé par l'acide chlorhydrique, l'acide bromhydrique et l'acide iodhydrique.

9. Procédé selon l'une quelconque des revendications 1 à 8, caractérisé en ce que la solution aqueuse acide d'ions halogénures contient des ions chlorure associés à des ions bromure et/ou iodure.

10. Procédé selon l'une quelconque des revendications 1 à 9, caractérisé en ce que la solution aqueuse contenant des ions halogénures est une solution aqueuse d'acide chlorhydrique et d'au moins un composé vecteur d'ions halogénures pris dans le groupe formé par les ions chlorure, bromure, iodure.

11. Procédé selon la revendication 10, caractérisé en ce que le vecteur d'ions halogénures est pris dans le groupe formé par l'acide bromhydrique, l'acide iodhydrique, les chlorures, bromures et iodures alcalins et alcalino-terreux, les chlorures, bromures et iodures d'ammonium, d'ammonium quaternaires, les chlorhydrates, bromhydrates et iodhydrates d'amines.

12. Procédé selon l'une quelconque des revendications 10 à 11, caractérisé en ce que la quantité de composé vecteur d'ions chlorures est telle que la concentration totale de la solution aqueuse acide en ions chlorure est d'au moins 8 ions-g/l.

13. Procédé selon l'une quelconque des revendications 10 à 11, caractérisé en ce que le composé vecteur d'ions halogénures est un composé vecteur d'ions iodures et bromures et que la concentration de la solution aqueuse d'acide chlorhydrique est au moins égale à 0,5 moles/l.

14. Procédé selon la revendication 13, caractérisé en ce que la quantité de composé vecteur d'ions iodures est telle que la concentration de la solution aqueuse d'acide chlorhydrique en ions iodures est comprise entre $1.10^{-6}$ et 1 ion $I^-$ par litre.

15. Procédé selon la revendication 13, caractérisé en ce que la quantité de composé vecteur d'ions bromures est telle que le concentration de la solution aqueuse d'acide chlorhydrique en ions bromures est comprise entre $1.10^{-2}$ et 10 ions $Br^-$ par litre.

16. Procédé selon la revendication 11, caractérisé en ce que le composé vecteur d'ions halogénures est pris dans le groupe des chlorures, bromures et iodures de sodium et de potassium.

17. Procédé selon l'une quelconque des revendications 1 à 16, caractérisé en ce que la température de la réaction est comprise entre 50 et 350°C.

18. Procédé selon l'une quelconque des revendications 1 à 17, caractérisé en ce que la pression partielle d'hydrogène est comprise entre 0,1 et 60 bars.

19. Procédé selon l'une quelconque des revendications 1 à 18, caractérisé en ce que le catalyseur est du palladium déposé sur un support inerte.

20. Procédé selon la revendication 19, caractérisé en ce que la quantité de catalyseur exprimée en poids de métal noble pour 100 g de polychlorophénol de formule (I) est comprise entre 0,01 g et 10 g.

21. Procédé selon l'une quelconque des revendications 1 à 20, caractérisé en ce que le polychlorophénol de formule (I) est pris dans le groupe formé par les dichloro- et trichlorophénols comportant un atome de chlore sur l'une au moins des positions méta par rapport à l'hydroxyle phénolique.

22. Procédé selon l'une des revendications 1 à 20, caractérisé en ce que le polychlorophénol de formule (I) est pris dans le groupe formé par les dichloro-3,4 phénol, dichloro-2,5 phénol et pentachlorophénol.

**Patentansprüche**

1. Verfahren zur selektiven Erzielung von Chlorphenolen mit einem Chloratom an wenigstens einer der meta-Stellungen in bezug auf das phenolische Hydroxyl durch Hydrierung in der Wärme in flüssiger Phase in Gegenwart eines Katalysators auf Basis eines Edelmetalls der Gruppe VIII des Periodensystems, von Chlorphenolen der allgemeinen Formel (I)

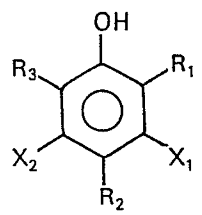

(I)

worin:

$X_1$ und $X_2$, die identisch oder verschieden sind, bedeuten ein Chloratom, ein Wasserstoffatom, einen Alkyl-, Aryl-, Arylalkyl-, Alkyloxy-, Aryloxyrest, wobei wenigstens eines der Symbole $X_1$ und $X_2$ ein Chloratom bedeutet,

$R_1$, $R_2$ und $R_3$, die identisch oder verschieden sind, bedeuten ein Chloratom, ein Wasserstoffatom, einen Alkylrest, einen Aryl-, Arylalkylrest, einen Alkyloxy-, Aryloxyrest, wobei wenigstens eines der Symbole $R_1$, $R_2$ und $R_3$ ein Chloratom bedeutet, dadurch gekennzeichnet, dass die Reaktion in einem sauren Lösungsmittelmilieu, das wenigstens teilweise wässerig ist und Halogenidionen enthält, durchgeführt wird.

2. Verfahren gemäss Anspruch 1, dadurch gekennzeichnet, dass das wenigstens teilweise wässerige Lösungsmittelmilieu aus Wasser oder aus einem Gemisch Wasser/organisches Lösungsmittel mit wenigstens 5 Vol.-% Wasser und vorzugsweise wenigstens 50 Vol.-% Wasser besteht.

3. Verfahren gemäss einem der Ansprüche 1 oder 2, dadurch gekennzeichnet, dass diejenigen der Reste $X_1$, $X_2$, $R_1$, $R_2$ und $R_3$, welche kein Chloratom bedeuten, einen Alkylrest mit 1 bis 10 Kohlenstoffatomen; einen Phenylrest; einen Benzylrest; einen Alkyloxyrest mit 1 bis 10 Kohlenstoffatomen; einen Phenoxyrest bedeuten.

4. Verfahren gemäss einem der Ansprüche 1 bis 3, dadurch gekennzeichnet, dass die Konzentration der sauren wässerigen Lösung an Protonen mindestens 0,5 Ion-g/l beträgt.

5. Verfahren gemäss einem der Ansprüche 1 bis 4, dadurch gekennzeichnet, dass die Konzentration der sauren wässerigen Lösung an Protonen höchstens gleich 15 Ionen-g/l beträgt.

6. Verfahren gemäss einem der Ansprüche 1 bis 5, dadurch gekennzeichnet, dass die Konzentration der sauren wässerigen Lösung an Halogenidionen wenigstens 2 Ionen-g/l beträgt.

7. Verfahren gemäss einem der Ansprüche 1 bis 6, dadurch gekennzeichnet, dass die Konzentration der sauren wässerigen Lösung an Halogenidionen höchstens gleich 15 Ionen-g/l beträgt.

8. Verfahren gemäss einem der Ansprüche 1 bis 7, dadurch gekennzeichnet, dass die Halogenidionen enthaltende, saure wässerige Lösung eine wässerige Lösung einer Wasserstoffsäure aus der Gruppe, gebildet von Chlorwasserstoffsäure, Bromwasserstoffsäure und Jodwasserstoffsäure, ist.

9. Verfahren gemäss einem der Ansprüche 1 bis 8, dadurch gekennzeichnet, dass die saure wässerige Lösung der Halogenidionen Chloridionen, assoziiert mit Bromid- und/oder Jodidionen, enthält.

10. Verfahren gemäss einem der Ansprüche 1 bis 9, dadurch gekennzeichnet, dass die Halogenidionen enthaltende, wässerige Lösung eine wässerige Lösung von Chlorwasserstoffsäure und wenigstens eine Halogenidionen aus der Gruppe, gebildet von den Chlorid-,Bromid-,Jodidionen liefernd Verbindung ist.

11. Verfahren gemäss Anspruch 10, dadurch gekennzeichnet, dass der Halogenidionenlieferant genommen wird aus der Gruppe, gebildet durch Bromwasserstoffsäure, Jodwasserstoffsäure, den Alkali- und Erdalkalichloriden, -bromiden und -jodiden, den Ammonium- und quaternären-Ammoniumchloriden, -bromiden und -jodiden, den Aminhydrochloriden, -hydrobromiden und -hydrojodiden.

12. Verfahren gemäss einem der Ansprüche 10 bis 11, dadurch gekennzeichnet, dass die Menge an Chloridionen liefernder Verbindung derart ist, dass die Gesamtkonzentration der sauren wässerigen Lösung an Chloridionen mindestens 8 Ionen-g/l beträgt.

13. Verfahren gemäss einem der Ansprüche 10 bis 11, dadurch gekennzeichnet, dass die Halogenidionen liefernde Verbindung eine Jodidionen und Bromidionen liefernde Verbindung ist, und dass die Konzentration der wässerigen Chlorwasserstoffsäurelösung mindestens 0,5 Mol/1 beträgt.

14. Verfahren gemäss Anspruch 13, dadurch gekennzeichnet, dass die Menge an Jodidionen liefernder Verbindung derart ist, dass die Konzentration der wässerigen Chlorwasserstoffsäurelösung an Jodidionen zwischen $1.10^{-6}$ und 1 Ion $J^-$ pro Liter beträgt.

15. Verfahren gemäss Anspruch 13, dadurch gekennzeichnet, dass die Menge an Bromidionen liefernder Verbindung derart ist, dass die Konzentration der wässerigen Chlorwasserstoffsäurelösung an Bromidionen zwischen $1.10^{-2}$ und 10 $Br^-$-Ionen pro Liter beträgt.

16. Verfahren gemäss Anspruch 11, dadurch gekennzeichnet, dass die Halogenidionen liefernde Verbindung aus der Gruppe von Natrium- und Kaliumchloriden, -bromiden und -jodiden genommen wird.

17. Verfahren gemäss einem der Ansprüche 1 bis 16, dadurch gekennzeichnet, dass die Reaktionstemperatur zwischen 50 und 350°C beträgt.

18. Verfahren gemäss einem der Ansprüche 1 bis 17, dadurch gekennzeichnet, dass der Wasserstoffpartialdruck zwischen 0,1 und 60 bar beträgt.

19. Verfahren gemäss einem der Ansprüche 1 bis 18, dadurch gekennzeichnet, dass der Katalysator Palladium, abgesetzt auf einem inerten Träger, ist.

20. Verfahren gemäss Anspruch 19, dadurch gekennzeichnet, dass die Katalysatormenge, ausgedrückt in Gewicht Edelmetall pro 100 g Polychlorphenol der Formel (I), zwischen 0,01 g und 10 g beträgt.

21. Verfahren gemäss einem der Ansprüche 1 bis 20, dadurch gekennzeichnet, dass das Polychlorphenol der Formel (I) genommen ist aus der Gruppe, gebildet von den Dichlor- und Trichlorphenolen mit einem Chloratom an wenigstens einer der meta-Stellungen im Bezug auf das phenolische Hydroxyl.

22. Verfahren gemäss einem der Ansprüche 1 bis 20, dadurch gekennzeichnet, dass das Polychlorphenol der Formel (I) aus der Gruppe, gebildet von 3,4-Dichlorphenol, 2,5-Dichlorphenol und Pentachlorphenol, genommen ist.

**Claims**

1. Process for selectively obtaining chlorophenols containing a chlorine atom in at least one of the metapositions relative to the phenolic hydroxyl, by the hydrogenation, under the action of heat, in the liquid phase, in the presence of a catalyst based on a noble metal of group VIII of the periodic classification, of polychlorophenols of the general formula (I):

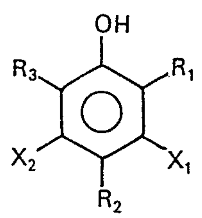

(I)

in which: $X_1$ and $X_2$, which are identical or different, represent a chlorine atom, a hydrogen atom or an alkyl, aryl, arylalkyl, alkoxy or aryloxy radical, at least one of the symbols $X_1$ and $X_2$ representing a chlorine atom, and $R_1$, $R_2$ and $R_3$, which are identical or different, represent a chlorine atom, a hydrogen atom, an alkyl radical, an aryl or arylalkyl radical or an alkoxy or aryloxy radical, at least one of the symbols $R_1$, $R_2$ and $R_3$ representing a chlorine atom, characterised in that the reaction is carried out in an acid solvent medium which is at least partially aqueous and which contains halide ions.

2. Process according to Claim 1, characterised in that the at least partially aqueous solvent medium consists of water or of water/organic solvent mixture containing at least 5 % by volume of water.

3. Process according to one of Claims 1 or 2, characterised in that those radicals $X_1$, $X_2$, $R_1$, $R_2$ and $R_3$ which do not symbolise a chlorine atom represent an alkyl radical containing from 1 to 10 carbon atoms, a phenyl radical, a benzyl radical, an alkoxy radical containing from 1 to 10 carbon atoms or a phenoxy radical.

4. Process according to any one of Claims 1 to 3, characterised in that the concentration of protons in the acid aqueous solution is at least 0.5 g ion/litre.

5. Process according to any one of Claims 1 to 4, characterised in that the concentration of protons in the acid aqueous solution is equal to at most 15 g ions/litre.

6. Process according to any one of Claims 1 to 5, characterised in that the concentration of halide ions in the acid aqueous solution is at least 2 g ions/litre.

7. Process according to any one of Claims 1 to 6, characterised in that the concentration of halide ions in the acid aqueous solution is equal to at most 15 g ions/litre.

8. Process according to any one of Claims 1 to 7, characterised in that the acid aqueous solution containing halide ions is an aqueous solution of a hydracid taken from the group comprising hydrochloric acid, hydrobromic acid and hydriodic acid.

9. Process according to any one of Claims 1 to 8, characterised in that the acid aqueous solution of halide ions contains chloride ions in combination with bromide and/or iodide ions.

10. Process according to any one of Claims 1 to 9, characterised in that the aqueous solution containing halide ions is an aqueous solution of hydrochloric acid and of at least one carrierof halide ions taken from the group comprising chloride, bromide and iodide ions.

11. Process according to Claim 10, characterised in that the halide ion carrier is taken from the group comprising hydrobromic acid, hydriodic acid, alkali metal and alkaline earth metal chlorides, bromides and iodides, ammonium and quaternary ammonium chlorides, bromides and iodides and amine hydrochlorides, hydrobromides and hydriodides.

12. Process according to either one of Claims 10 and 11, characterised in that the amount of chloride ion carrier is such that the total concentration of chloride ions in the acid aqueous solution is at least 8 g ions/litre.

13. Process according to either one of Claims 10 and 11, characterised in that the halide ion carrier is a carrier of iodide and bromide ions, and in that the concentration of the aqueous solution of hydrochloric acid is equal to at least 0.5 mol/litre.

14. Process according to Claim 13, characterised in that the amount of iodide ion carrier is such that the concentration of iodide ions in the aqueous solution of hydrochloric acid is between $1.10^{-6}$ and 1 $I^-$ ion per litre.

15. Process according to Claim 13, characterised in that the amount of bromide ion carrier is such that the concentration of bromide ions in the aqueous solution of hydrochloric acid is between $1.10^{-2}$ and 10 $Br^-$ ions per litre.

16. Process according to Claim 11, characterised in that the halide ion carrier is taken from the group comprising the chlorides, bromides and iodides of sodium and potassium.

17. Process according to any one of Claims 1 to 16, characterised in that the reaction temperature is between 50 and 350°C.

18. Process according to any one of Claims 1 to 17, characterised in that the partial hydrogen pressure is between 0.1 and 60 bars.

19. Process according to any one of Claims 1 to 18, characterised in that the catalyst is palladium deposited on an inert support.

20. Process according to Claim 19, characterised in that the amount of catalyst, expressed as the weight of noble metal per 100 g of polychlorophenol of the formula (I), is between 0.01 g and 10 g.

21. Process according to any one of Claims 1 to 20, characterised in that the polychlorophenol of the formula (I) is taken from the group comprising the dichlorophenols and trichlorophenols containing a chlorine atom in at least one of the meta-positions relative to the phenolic hydroxyl.

22. Process according to Claim 21, characterised in that the polychlorophenol of the formula (I) is taken from the group comprising 3,4-dichlorophenol, 2,5-dichlorophenol and pentachlorophenol.